(19) Europäisches Patentamt
European Patent Office
Office européen des brevets

(11) Veröffentlichungsnummer: 0 224 809
A1

(12) EUROPÄISCHE PATENTANMELDUNG

(21) Anmeldenummer: 86116122.2

(22) Anmeldetag: 21.11.86

(51) Int. Cl.⁴: C 07 D 513/04
A 61 K 31/54
//(C07D513/04, 285:00, 235:00)

(30) Priorität: 03.12.85 DE 3542662

(43) Veröffentlichungstag der Anmeldung:
10.06.87 Patentblatt 87/24

(84) Benannte Vertragsstaaten:
AT BE CH DE ES FR GB IT LI NL SE

(71) Anmelder: BAYER AG
Konzernverwaltung RP Patentabteilung
D-5090 Leverkusen 1 Bayerwerk(DE)

(72) Erfinder: Meyer, Horst, Dr.
Henselweg 11
D-5600 Wuppertal 1(DE)

(72) Erfinder: Hirth, Claudia, Dr.
Stockmannsmühle 127
D-5600 Wuppertal 1(DE)

(54) **Imidazothiadiazinalkensäureamide, Verfahren zu ihrer Herstellung, Zwischenprodukte zu ihrer Herstellung und ihre Verwendung zur Behandlung von Krankheiten.**

(57) Die Erfindung betrifft Imidazothiadiazinalkencarbonsäureamide der allgemeinen Formel (I)

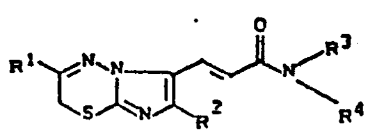

(I)

mit den in der Beschreibung angegebenen Substituentendefinitionen, Verfahren zu ihrer Herstellung, Zwischenprodukte zu ihrer Herstellung und ihre Verwendung zur Behandlung von Krankheiten, insbesondere in antihypertensiv und diuretisch wirksamen Arzneimitteln.

EP 0 224 809 A1

BAYER AKTIENGESELLSCHAFT
Konzernverwaltung RP
Patentabteilung

5090 Leverkusen, Bayerwerk

Si-klu/c

# Imidazothiadiazinalkensäureamide

Die Erfindung betrifft Imidazothiadiazinalkensäureamide, Verfahren zur ihrer Herstellung, Zwischenprodukte zu ihrer Herstellung und ihre Verwendung zur Behandlung von Krankheiten, insbesondere in antihypertensiv und diuretisch wirksamen Arzneimitteln.

Aus US-Patentschrift 4.444.770 ist bekannt, daß Imidazothiadiazolalkencarbonsäureamide diuretische Wirkung haben.

Die vorliegende Erfindung betrifft Imidazothiadiazinalkensäureamide der allgemeinen Formel (I)

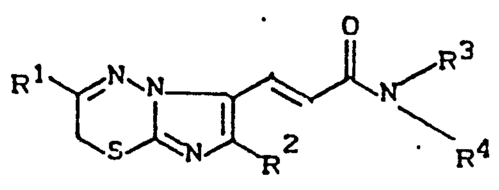

(I),

in welcher

$R^1$
- für Wasserstoff oder
- für Alkoxy oder
- für gegebenenfalls durch Phenyl, Cyano, Hydroxy oder Halogen substituiertes Alkyl oder Alkenyl steht, wobei die Kohlenstoffkette des Alkylrestes gegebenenfalls durch O, S, NH, N-Alkyl, N-Aryl oder N-Aralkyl unterbrochen ist, oder
- für einen Aryl- oder Heterocyclylrest steht, der gegebenenfalls ein, zwei oder drei gleiche oder verschiedene Substituenten aus der Gruppe Alkyl, Alkoxy, Alkylthio, Trifluormethyl, Trifluormethoxy, Halogen, Nitro, Cyano oder Alkylsulfonyl trägt, oder
- für ein Gruppe $SO_n$-Alkyl steht, worin n 0, 1 oder 2 bedeutet, oder
- für eine Gruppe der Formel

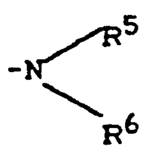 oder 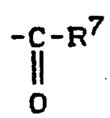 steht,

wobei

$R^5$, $R^6$ - gleich oder verschieden sind und für Wasserstoff, oder
- für gegebenenfalls durch O, S, NH oder N-Alkyl in der Kette unterbrochenes Alkyl stehen, oder
- für Aryl oder Aralkyl stehen,

oder

$R^5$ und $R^6$ gemeinsam mit dem Stickstoffatom einen 5 bis 7-gliedrigen Ring bilden, der gegebenenfalls ein oder zwei Sauerstoff-, Schwefel- und/oder Stickstoffatome als

weitere Heteroatome enthalten kann, und wobei diese Stickstoffatome gegebenenfalls durch Alkyl, Aryl oder Aralkyl substituiert sind,

und wobei

$R^7$ - für Wasserstoff,
- für Hydroxy,
- für Alkyl oder Alkenyl,
- für Alkoxy oder Alkenyloxy,
- für Aryloxy oder Aralkoxy steht, oder
- für eine Gruppe der Formel

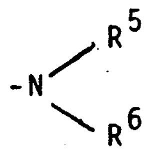 steht,

wobei $R^5$ und $R^6$ die bereits angegebene Bedeutung haben,

$R^2$ - für Aryl steht, das gegebenenfalls bis zu dreifach gleich oder verschieden substituiert ist durch Halogen, Alkyl, Alkoxy, Alkylthio, Nitro, Cyano oder Trifluormethyl, oder
- für gegebenenfalls durch Halogen, Alkyl, Alkoxy oder Trifluormethyl substituiertes Pyridyl, Thienyl, Furyl, Pyrimidyl, Pyridazinyl, Chinolyl oder Isochinolyl steht,

und

$R^3$, $R^4$ - gleich oder verschieden sind und
- für Wasserstoff,
- für Aryl, oder
- für einen geradkettigen, verzweigten oder cyclischen, gesättigten oder ungesättigten

Kohlenwasserstoffrest mit bis zu 10 C-Atomen stehen der gegebenenfalls durch O, S, NH, N-Alkyl, N-Aryl oder N-Aralkyl unterbrochen ist, und der gegebenenfalls substituiert ist durch Hydroxy, Alkoxy, Trifluormethyl, Halogen, Phenyl, Alkoxycarbonyl oder Dialkylamino, wobei die beiden Alkylreste gegebenenfalls gemeinsam mit dem Stickstoffatom einen 5-7-gliedrigen Ring bilden, der gegebenenfalls durch O, S, NH oder N-Alkyl unterbrochen ist und wobei die vorgenannten Alkyl- sowie Phenylreste ihrerseits gegebenenfalls durch Halogen, Trifluormethyl, Alkyl, Aryl, Aralkyl, Alkoxy, Alkylthio, Alkylsulfonyl oder Cyano substituiert sind,

oder

$R^3$ und $R^4$ gemeinsam mit dem Stickstoffatom einen 3- bis 8-gliedrigen, gesättigten oder ungesättigten Ring bilden, der gegebenenfalls ein oder zwei O, S, N, NH, N-Alkyl, N-Aryl oder N-Aralkyl enthält, der gegebenenfalls bis zu drei Substituenten aus der Reihe Alkyl, Halogen, Aryl, Aralkyl, Alkoxycarbonyl, Hydroxyalkyl, Alkoxyalkyl oder Trifluormethyl tragen kann, oder der mit einem gegebenenfalls substituierten aromatischen Ring kondensiert sein kann,

sowie ihre physiologisch unbedenklichen Salze, sowie ihre stereoisomeren Formen in Form von Enantiomeren, Diastereomeren und E/Z-Isomeren.

Die erfindungsgemäßen Imidazothiadiazinalkensäureamide haben gute diuretische sowie saluretische Wirkung.

Im Rahmen der obigen Substituentendefinition steht Alkyl im allgemeinen für einen geradkettigen, verzweigten oder cyclischen gesättigten Kohlenwasserstoff mit bis zu 10 C-Atomen, bevorzugt mit bis zu 8 C-Atomen.

Alkenyl steht im allgemeinen für einen geradkettigen, verzweigten oder cyclischen Kohlenwasserstoffrest mit bis zu 10 C-Atomen und bis zu zwei Doppelbindungen, bevorzugt mit bis zu 8 C-Atomen und einer Doppelbindung.

Alkoxy steht im allgemeinen für einen geradkettigen oder verzweigten Alkylrest mit bis zu 10 C-Atomen, bevorzugt mit bis zu 8 C-Atomen, der über ein Sauerstoffatom gebunden ist.

Aryl steht im allgemeinen für einen aromatischen Kohlenwasserstoffrest mit bis zu 14 C-Atomen, bevorzugt für Phenyl oder Naphthyl.

Aralkyl steht im allgemeinen für einen Phenylrest, der über eine Alkylkette mit bis zu 6 C-Atomen gebunden ist. Bevorzugt steht Aralkyl für Benzyl oder Phenethyl.

Heterocyclyl steht im allgemeinen für eine 5- bis 7-gliedrigen Ring, der als Heteroatome bis zu 3 Sauerstoff-, Schwefel- und/oder Stickstoffatome enthalten kann, der gesättigt oder ungesättigt sein kann und der gegebenenfalls durch Halogen, Hydroxy, Amino, Alkylamino, Dialkylamino, Alkyl, Trifluormethyl oder Alkoxy substituiert sein kann. Bevorzugt steht Heterocyclyl für Reste wie Pyridyl, Thienyl, Furyl, Pyrrolyl, Pyrimidyl, Pyrazinyl, Pyridazinyl, Chinolyl, Isochinolyl, Piperidinyl, Piperazinyl, Morpholinyl oder Thiomorpholinyl.

Halogen steht im allgemeinen für Fluor, Chlor, Brom oder Iod, bevorzugt für Fluor, Chlor oder Brom.

Bevorzugte Imidazothiadiazinalkensäureamide der allgemeinen Formel I sind solche, in denen

$R^1$
- für Wasserstoff oder
- für $C_1$-$C_6$-Alkoxy steht, oder
- für gegebenenfalls durch Phenyl, Cyano, Hydroxy, Fluor, Chlor oder Brom substituiertes $C_1$-$C_6$-Alkyl, $C_2$-$C_6$-Alkenyl, Cyclopentyl oder Cyclohexyl steht, wobei die Kohlenstoffkette des Alkylrestes gegebenenfalls durch O, S, NH, N-$C_1$-$C_6$-Alkyl, N-Phenyl oder N-Benzyl unterbrochen ist,
  oder
- für einen Phenyl-, Naphthyl-, Furyl-, Thienyl-, Pyrimidyl-, Pyrazinyl-, Pyridyl-, Chinolyl- oder Isochinolylrest steht, der gegebenenfalls durch ein oder zwei gleiche oder verschiedene Substituenten der Reihe $C_1$-$C_6$-Alkyl, $C_1$-$C_6$-Alkoxy, Fluor, Chlor, Brom, Nitro, Cyano, Trifluormethyl, Trifluormethoxy oder $C_1$-$C_4$-Alkylsulfonyl substituiert ist, oder
- für eine Gruppe $SO_n$-Alkyl steht,

wobei

n für 0 oder 2 steht und Alkyl bis zu 6 C-Atome hat,

oder

- für eine Gruppe der Formel

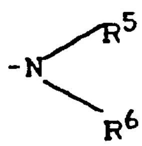 oder 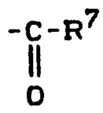 steht,

wobei

$R^5$, $R^6$ - gleich oder verschieden sind und

- für Wasserstoff stehen, oder
- für $C_1$-$C_6$-Alkyl stehen, das gegebenenfalls durch O, S, NH oder N-$C_1$-$C_6$-Alkyl unterbrochen ist, oder
- für Phenyl, Naphthyl oder Benzyl stehen,

oder

$R^5$ und $R^6$ gemeinsam mit dem Stickstoffatom einen 5 bis 7-gliedrigen Ring bilden, der gegebenenfalls ein oder zwei Sauerstoff-, Schwefel- und/oder Stickstoffatome als weitere Heteroatome enthalten kann und wobei diese Stickstoffatome gegebenenfalls durch $C_1$-$C_6$-Alkyl, Phenyl oder Benzyl substituiert sind,

und wobei

$R^7$ - für Wasserstoff,

- für Hydroxy,

- für $C_1$-$C_6$-Alkyl, $C_2$-$C_6$-Alkenyl,
- für $C_1$-$C_6$-Alkoxy, $C_2$-$C_6$-Alkenyloxy,
- für Phenoxy oder Benzyloxy steht, oder
- für eine Gruppe der Formel

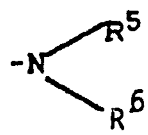

steht,

wobei $R^5$ und $R^6$ die bereits angegebene Bedeutung haben,

$R^2$ - für gegebenenfalls durch Fluor, Chlor, Brom, $C_1$-$C_6$-Alkyl, $C_1$-$C_6$-Alkoxy, Nitro, Cyano oder Trifluormethyl substituiertes Phenyl steht, oder
- für gegebenenfalls durch Fluor, Chlor, Brom oder $C_1$-$C_4$-Alkyl substituiertes Pyridyl, Thienyl, Furyl, oder Pyrimidyl steht,

und

$R^3$, $R^4$ - gleich oder verschieden sind und
- für Wasserstoff,
- für Phenyl oder
- für einen geradkettigen, verzweigten oder cyclischen Kohlenwasserstoffrest mit bis zu 8 C-Atomen stehen, der gegebenenfalls durch O, S, NH, N-$C_1$-$C_6$-Alkyl, N-Phenyl oder N-Benzyl unterbrochen ist, und der gegebenenfalls substituiert ist durch Hydroxy, $C_1$-$C_6$-Alkoxy, Trifluormethyl, Fluor, Chlor, Brom, Phenyl, $C_1$-$C_6$-Alkoxycarbonyl, Di-$C_1$-$C_6$-Alkylamino, Piperidin, Piperazin, Morpholin, Thiomorpholin, N-Methylpiperazin oder

Pyrrolidin, wobei die vorgenannten Alkyl- sowie Phenylreste ihrerseits gegebenenfalls durch Fluor, Chlor, Brom, Trifluormethyl, $C_1$-$C_6$-Alkyl, Phenyl, Benzyl, $C_1$-$C_6$-Alkoxy, $C_1$-$C_6$-Alkylsulfonyl oder Cyano substituiert sind,

oder

$R^3$ und $R^4$ gemeinsam mit dem Stickstoffatom einen 5- bis 7-gliedrigen Ring bilden, der gegebenenfalls 1 oder 2 weitere Heteroatome aus der Gruppe Sauerstoff, Schwefel oder Stickstoff enthält, wobei der Stickstoff gegebenenfalls durch Wasserstoff, $C_1$-$C_6$-Alkyl, Phenyl oder Benzyl substituiert ist und wobei der Ring durch bis zu drei gleiche oder verschiedene Substituenten aus der Reihe $C_1$-$C_6$-Alkyl, Fluor, Chlor, Brom, Phenyl, Benzyl, $C_1$-$C_6$-Alkoxycarbonyl, Hydroxy-$C_1$-$C_6$-alkyl, $C_1$-$C_4$-Alkoxy-$C_1$-$C_6$-alkyl oder Trifluormethyl substituiert sein kann, oder wobei der Ring mit einem gegebenenfalls durch Fluor, Chlor, Brom oder Nitro substituierten aromatischen Ring kondensiert sein kann,

sowie ihre physiologisch unbedenklichen Salze, sowie ihre stereoisomeren Formen in Form von Enantiomeren, Diastereomeren und E/Z-Isomeren.

Besonders bevorzugt sind Verbindungen der allgemeinen Formel I in welcher

$R^1$ - für Wasserstoff,

- für geradkettiges oder verzweigtes $C_1$-$C_4$-Alkoxy oder

- für gegebenenfalls durch Fluor, Chlor oder Hydroxy substituiertes geradkettiges oder verzweigtes $C_1$-$C_6$-Alkyl steht, wobei die Alkylkette gegebenenfalls durch O, S, NH, N-$C_1$-$C_4$-Alkyl, N-Phenyl oder N-Benzyl unterbrochen ist,

  oder
- für einen Phenyl-, Naphthyl-, Furyl-, Thienyl-, Pyrimidyl- oder Pyridylrest steht, oder
- für eine Gruppe der Formel

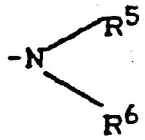 oder 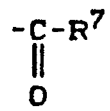 steht,

wobei

$R^5$, $R^6$ - gleich oder verschieden sind und
- für Wasserstoff oder
- für geradkettiges oder verzweigtes $C_1$-$C_6$-Alkyl stehen, das gegebenenfalls durch O, S, NH oder N-$C_1$-$C_3$-Alkyl unterbrochen ist,

  oder
- für Phenyl oder Benzyl stehen

oder

$R^5$ und $R^6$ gemeinsam mit dem Stickstoffatom einen 5- bis 6-gliedrigen Ring bilden, der ein Sauerstoff-, Schwefel-und/oder Stickstoffatom als weiteres Heteroatom enthalten kann, und wobei dieses Stickstoffatom gegebenen-

falls durch $C_1$-$C_3$-Alkyl, Phenyl oder Benzyl substituiert ist,

und wobei

$R^7$ - für Wasserstoff,
- für Hydroxy,
- für geradkettiges oder verzweigtes $C_1$-$C_4$-Alkyl,
- für geradkettiges oder verzweigtes $C_1$-$C_4$-Alkoxy oder
- für Phenoxy oder Benzyloxy steht,

$R^2$ - für gegebenenfalls durch Chlor, Brom oder $C_1$-$C_4$-Alkyl substituiertes Phenyl, oder
- für Thienyl steht,

und

$R^3$, $R^4$ - gleich oder verschieden sind und
- für Wasserstoff,
- für Phenyl oder
- für einen geradkettigen, verzweigten oder cyclischen Kohlenwasserstoffrest mit bis zu 6 C-Atomen stehen, der gegebenenfalls durch O, S, NH, N-$C_1$-$C_4$-Alkyl, N-Phenyl oder N-Benzyl unterbrochen ist, und der gegebenenfalls substituiert ist durch Hydroxy, $C_1$-$C_4$-Alkoxy, Trifluormethyl, Fluor, Chlor, Phenyl, Di-$C_1$-$C_4$-alkylamino, Piperidin, Piperazin, N-Methylpiperazin, Morpholin oder Pyrrolidin, wobei die vorgenannten Alkyl- oder Phenylreste ihrerseits gegebenenfalls durch Fluor, Chlor, Trifluormethyl, $C_1$-$C_4$-Alkyl oder $C_1$-$C_4$-Alkoxy substituiert sind,

oder

$R^3$ und $R^4$ gemeinsam mit dem Stickstoffatom einen 5- bis 7-gliedrigen Ring bilden, der gegebenenfalls ein oder zwei weitere Heteroatome aus der Gruppe Sauerstoff, Schwefel oder Stickstoff enthält, wobei dieser Stickstoff gegebenenfalls durch Wasserstoff, $C_1$-$C_4$-Alkyl, Phenyl oder Benzyl substituiert ist und wobei der Ring durch bis zu zwei gleiche oder verschiedene Substituenten aus der Reihe $C_1$-$C_4$-Alkyl, Fluor, Chlor, $C_1$-$C_4$-Hydroxyalkyl, $C_1$-$C_4$-Alkoxy-$C_1$-$C_4$-alkyl oder Trifluormethyl substituiert sein kann oder wobei der Ring mit einem aromatischen Ring kondensiert sein kann,

sowie ihre physiologisch unbedenklichen Salze, sowie ihre stereoisomeren Formen in Form von Enantiomeren, Diastereomeren und E/Z-Isomeren.

Je nach Wahl der Ausgangssubstanzen können die erfindungsgemäßen Verbindungen in stereoisomeren Formen existieren, die sich entweder wie Bild und Spiegelbild (Enantiomere) oder die sich nicht wie Bild und Spiegelbild (Diastereomere) verhalten. Sowohl die Antipoden als auch die Racemformen als auch die Diastereomerengemische sind Gegenstand der vorliegenden Erfindung. Die Racemformen lassen sich ebenso wie die Diastereomeren in bekannter Weise in die stereoisomer einheitlichen Bestandteile trennen (Vgl. z.B. E.L. Eliel, Stereochemistry of Carbon Compounds (McGraw Hill, 1962).

Darüber hinaus werden die durch die Doppelbinding der Seitenkette möglichen Z/E-Isomeren beansprucht, die nach bekannten Verfahren hergestellt oder nach bekannten Verfahren ineinander überführt werden können.

Die erfindungsgemäßen Verbindungen können in Form ihrer Salze vorliegen. Im allgemeinen sind dies Salze der erfindungsgemäßen Stoffe mit anorganischen oder organischen Säuren. Bevorzugt sind jedoch die physiologisch unbedenklichen Salze der erfindungsgemäßen Stoffe mit anorganischen und organischen Säuren. Als Beispiele seinen genannt: Hydrohalogenide, Hydrogensulfate, Sulfate, Hydrogenphosphate, Acetate, Maleate, Citrate, Fumarate, Tartrate, Lactate oder Benzoate.

Die erfindungsgemäßen Verbindungen der allgemeinen Formel (I) erhält man, wenn man

[A] Aldehyde der allgemeinen Formel (II)

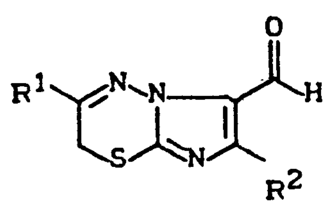

(II),

in welcher
$R^1$, $R^2$ die bereits angegebene Bedeutung haben,
mit Phosphonaten der allgemeinen Formel (III)

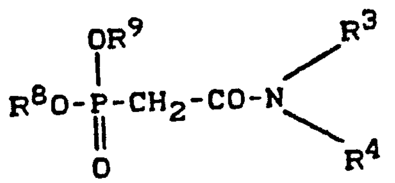 (III),

in welcher

$R^3$, $R^4$ die bereits angegebene Bedeutung haben,

$R^8$, $R^9$ für gegebenenfalls substituiertes Alkyl oder Aralkyl stehen

in Gegenwart von starken Basen in inerten organischen Lösungsmitteln bei Temperaturen von -20° C bis +150° C, bevorzugt von -20° C bis +110° C umsetzt,

oder wenn man

[B] Aldehyde der allgemeinen Formel (II) mit Acetamiden der allgemeinen Formel (IV)

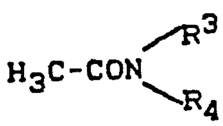 (IV),

in welcher

$R^3$, $R^4$ die bereits angegebene Bedeutung haben,

in Gegenwart saurer oder basicher Katalysatoren, gegebenenfalls in Anwesenheit inerter organischer Lösungsmittel bei Temperaturen zwischen 0° C und +200° C, bevorzugt zwischen +20° C und +150° C umsetzt,

oder indem man

 

[C] Alkencarbonsäuren der allgemeinen Formel (V)

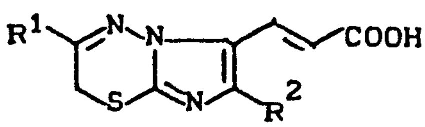

(V)

in welcher

$R^1$, $R^2$ die bereits angegebene Bedeutung haben, mit Aminen der allgemeinen Formel (VI)

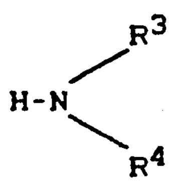

(VI),

in welcher

$R^3$, $R^4$ die bereits angegebene Bedeutung haben, gegebenenfalls nach Aktivierung der Carboxylgruppe über das entsprechende Säurechlorid in üblicher Weise in inerten organischen Lösungsmitteln bei Temperaturen zwischen 0° C und + 150 °C, bevorzugt zwischen + 20 °C und + 100 °C umsetzt.

Die als Ausgangsverbindungen eingesetzten Aldehyde der allgemeinen Formel (II) sind neu. Sie können nach bekannten Methoden hergestellt werden, indem man Imidazothiadiazine der allgemeinen Formel (VII)

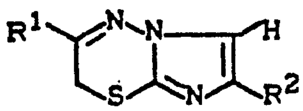 (VII),

in welcher

$R^1$ und $R^2$ die angegebene Bedeutung haben, mit Phosphoroxychlorid und Dimethylformamid bei Temperaturen von 0° C bis + 150 °C umsetzt, wie es beispielsweise in GB-Patentschrift 123.586, US-Patentschrift 3.809.691 oder US-Patentschrift 4.444.770 beschrieben wird.

Die als Ausgangsstoffe eingesetzten Phosphonate der Formel (III) sind bekannt oder können nach bekannten Methoden hergestellt werden [I. Shahek et al.;Isr. J. Chem. 7, 585 (1969)].

Die als Ausgangsstoffe verwendeten Acetamide der Formel (IV) [Verfahren A] sind bekannt oder können nach bekannten Methoden hergestellt werden [GB-Patentschrift 715 896 (1954); DBP 1.142.859 (1960)].

Die als Ausgangsstoffe eingesetzten Alkencarbonsäuren der Formel (V) sind neu. Sie können nach bekannten Verfahren hergestellt werden, indem man

[X] Carbonylverbindungen der allgemeinen Formel (II) mit Phosphonaten der allgemeinen Formel (VIII)

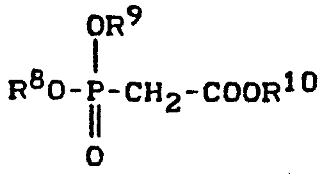 (VIII),

in welcher

$R^8$, $R^9$ - die bereits angegebene Bedeutung haben, und
$R^{10}$ - für Wasserstoff, Trialkylsilyl, Triarylsilyl oder für Alkyl oder Aralkyl steht,

in Gegenwart starker Basen in inerten organischen Lösungsmitteln zunächst zu den entsprechenden Alkencarbonsäurederivaten umsetzt und diese dann mit Säuren oder Basen zu den freien Carbonsäuren verseift, wie es von W.S. Wadsworth et al. in J. Am. Chem. Soc. 83, 1733 (1961) beschrieben wird,

oder indem man

[Y] Aldehyde der allgemeinen Formel (II) mit Malonsäure ($H_2C(COOH)_2$) oder mit Meldrumsäure in Gegenwart inerter organischer Lösungsmittel, gegebenenfalls in Gegenwart von Kondensationsmitteln umsetzt, wie es beispielsweise von G. Jones in Organic Reaction 15, 204 ff. beschrieben wird.

Die als Ausgangsverbindungen eingesetzten Thiadiazine der Formel VII sind bekannt oder können nach bekannten Methoden hergestellt werden [E. Bulka, W.-D. Pfeiffer, Z. Chem. 15, 482 (1975)].

Die als Ausgangsverbindungen eingesetzten Phosphonate der Formel VIII sind bekannt [J. Bontagy, R. Thomas, Chem. Rev. 74, 87 (1974)].

Als starke Basen bei der Durchführung der Verfahren [A] bzw. [X] können die üblichen starken Basen verwendet werden. Hierzu gehören bevorzugt Alkalihydride wie z.B. Natriumhydrid oder Kaliumhydrid, Alkalialkoholate wie z.B. Natriummethylat, Kaliummethylat, Natriumethylat, Kaliumethylat oder Kalium-tert.-butylat, oder Alkalimetallalkyle wie Methyllithium , Butyllithium oder Phenyllithium, oder Lithiumdiisopropylamid.

Die bei der Durchführung des Verfahrens [B] eingesetzen sauren oder basischen Katalysatoren sind bevorzugt organische Amine wie beispielsweise Trialkylamine (Triethylamin), Dialkylamine (Diisopropylamin), Pyridin, Piperidin, N-Methylpiperidin oder auch Gemische der genannten Amine, sowie anorganische Säuren wie Schwefelsäure oder Halogenwasserstoffsäuren, insbesondere Salzsäure, oder Carbonsäureanhydride wie Acetanhydrid.

Die für die Umsetzung mit Aminen (VI) zweckmäßige Aktivierung der freien Carboxylgruppe der Carbonsäuren (V) (Verfahren C) erfolgt vorzugsweise über das entsprechende Carbonsäurechlorid unter Verwendung von Halogenidbildnern wie Thionylchlorid, Phosphortrichlorid oder Phosphorpentachlorid.

Die bei der Durchführung des Verfahrens [Y] verwendeten Kondensationsmittel sind vorzugsweise Pyridin, Pyridinderivate wie Dialkylaminopyridine, Chinolin, Isochinolin,

Dialkylamine wie Diisopropylamin oder Dibutylamin, Triethylamin, Pyrrolidin, Piperidin oder N-Methylpiperidin sowie ähnliche stickstoffhaltige organische Basen.

Bei allen Verfahren können als Lösungsmittel die üblichen inerten organischen Lösungsmittel verwendet werden, die sich unter den Versuchsbedingungen nicht verändern.

Hierzu gehören vorzugsweie Ether wie Diethylether, Tetrahydrofuran, Dioxan, Glykolmonomethylether, Glykoldimethylether, oder Alkohole wie Methanol, Ethanol, Propanol, Butanol oder Benzylalkohol, Dimethylsulfoxid, Dimethylformamid, Hexamethylphosphorsäuretriamid, oder Kohlenwasserstoffe wie Benzol, Toluol, Xylol oder Erdölfraktionen, oder Basen wie Pyridin, Chinolin oder Picolin. Ebenso ist es möglich, Gemische der genannten Lösungsmittel zu verwenden.

Die erfindungsgemäßen Verbindungen zeichnen sich überraschenderweise durch starke biologische Wirkungen aus. Insbesondere besitzen sie ausgeprägte diuretische und saluretische Wirkungen und können daher als Diuretika, Saluretika und Antihypertensiva verwendet werden. Bei Tierversuchen an Ratten, zeigt sich, daß die erfindungsgemäßen Verbindungen bei oraler Applikation bereits bei geringen Dosierungen eine ausgeprägte diuretische und saluretische Wirkung bei gleichzeitiger guter Verträglichkeit besitzen.

Die pharmakologische Wirkung der erfindungsgemäßen Verbindungen wurden nach folgender Testmethode ermittelt: Nüchterne männliche Ratten im Gewicht von 150 bis 250 g (SPF, Wistar, je n=4 Paare) werden mit 10 ml/kg p.o. Tylose-Suspension (0,5%) als Kontrollen bzw. mit 100 mg/kg p.o. Prüfsubstanz in 10 ml/kg p.o. Tylose-Suspension mittels Schlundsonde behandelt. Die Tiere werden in Stoffwechselkäfige gebracht und die Harn- und Elektolytausscheidungen über 6 Stunden bestimmt ($Na^+$ und $K^+$ Bestimmung: IL-Flammenphotometer).

Die neuen erfindungsgemäßen Verbindungen sind als Arzneimittel verwendbare Substanzen. Sie bewirken bei oraler oder parenteraler Anwendung eine Steigerung der Wasser- und Salzausscheidung und können daher zur Behandlung oedematöser und hypertoner Zustände und zur Ausschwemmung toxischer Substanzen dienen.

Darüber hinaus können die Verbindungen bei akutem Nierenversagen eingesetzt werden. Insbesondere zeigen sie auch eine vorteilhafte uricosurische Wirkung.

Die neuen Verbindungen können in bekannter Weise in die üblichen Formulierungen übergeführt werden, wie Tabletten, Kapseln, Dragees, Pillen, Granulate, Sirupe, Emulsionen, Suspensionen und Lösungen, unter Verwendung inerter, nichttoxischer, pharmazeutisch geeigneter Trägersubstanzen oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den unten angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral oder parenteral.

Für den Fall der parenteralen Anwendung können Lösungen der Wirkstoffe unter Verwendung geeigneter flüssiger Trägermaterialien eingesetzt werden. Als besonders vorteilhaft für den Fall der parenteralen Anwendung hat sich die Tatsache herausgestellt, daß die erfindungsgemäßen Verbindungen in Wasser gut lösliche Salze zu bilden vermögen. Derartige Salze können auch für die orale Anwendung der erfindungsgemäßen Verbindungen eine erhöhte Bedeutung besitzen, indem sie die Resorption je nach Wunsch beschleunigen oder verzögern.

Im allgemeinen hat es sich als vorteilhaft erwiesen, bei parenteraler Applikation Mengen von etwa 0,05 bis 100 mg/kg, vorzugsweise etwa 0,1 bis 10 mg/kg Körpergewicht pro Tag zur Erzielung wirksamer Ergebnisse zu verabreichen. Bei oraler Applikation beträgt die Dosierung etwa 0,1 bis 500 mg/kg, vorzugsweise 0,5 bis 100 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen.

## Herstellungsbeispiele

### Beispiel 1

6-Formyl-3-methyl-7-phenyl-2H-imidazo[2,1-b]-1,3,4-thiadiazin

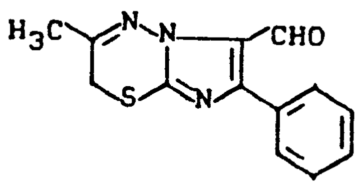

Zu 30 ml absolutem Dimethylformamid werden unter Rühren bei -10° C zunächst 18,4 g $POCl_3$ und anschließend weitere 70 ml Dimethylformamid zugetropft. Danach werden nach und nach unter Rühren 20 g 3-Methyl-7-phenyl-2H-imidazo/2,1-b7-1,3,4-thiadiazin eingetragen, 15 min. bei Raumtemperatur nachgerührt und dann 2 h auf 80° C erwärmt. Nach Abkühlen wird die Reaktionsmischung mit Wasser versetzt, der Niederschlag abgesaugt, mit Wasser gewaschen und getrocknet.

Ausbeute: 78 % der Theorie

Schmelzpunkt: 178° C.

### Beispiel 2

2-(3-Methyl-7-phenyl-2H-imidazo[2,1-b]-1,3,4-thiadiazin-6-yl)propensäure

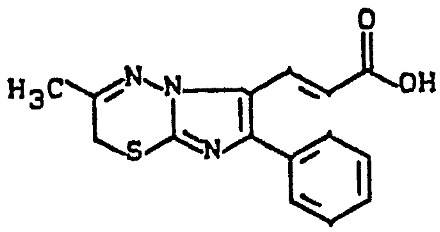

5,14 g 6-Formyl-3-methyl-7-phenyl-imidazo[2,1-b]-1,3,4-thiadiazin werden mit 3,12 g Malonsäure und 0,5 ml Piperidin in 6 ml Pyridin 10 h unter Rückfluß erhitzt. Nach Abkühlen wird mit Wasser versetzt, angesäuert und 12 h bei Raumtemperatur gerührt. Danach wird der Niederschlag abgesaugt, mit $NaHCO_3$-Lösung und 1 N NaOH versetzt, 30 min gerührt und vom Ungelösten abgesaugt. Das Filtrat wird angesäuert, der ausgefallene Feststoff abgesaugt und getrocknet.
Ausbeute: 65 % der Theorie
Schmelzpunkt: 240°C (Zers.).

Beispiel 3

(3-Methyl-7-phenyl-imidazo[2,1-b]-1,3,4-thiadiazin-6-yl)-propensäuremorpholinid

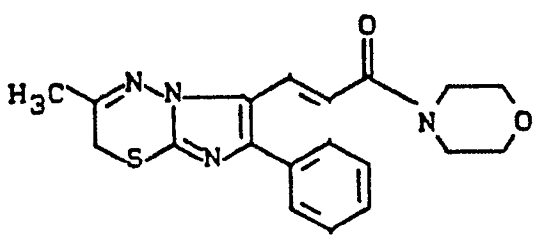

Zu 30 ml Dimethoxyethan werden 0,4 g NaH (80 %ig in Öl) gegeben. Danach tropft man 3,0 g Diethylphosphonoessigsäuremorpholinid zu, wobei die Temperatur bei 20° C gehalten wird. Nach 1 h Rühren bei Raumtemperatur gibt man weitere 20 ml Dimethoxyethan und portionsweise 2,5 g 6-Formyl-3-methyl-7-phenyl-imidazo[2,1-b]-1,3,4-thiadiazin zu, rührt 1 h bei Raumtemperatur und erhitzt dann 1 h im Rückfluß. dann kühlt man ab, engt die Lösung ein und reinigt das Produkt über eine Kieselgelsäure.
Ausbeute: 16 % der Theorie
Schmelzpunkt: 202° C.

Beispiel 4

(3-Methyl-7-phenyl-imidazo[2,1-b]-1,3,4-thiadiazin-6-yl)-propensäure-(4-methylpiperazid)

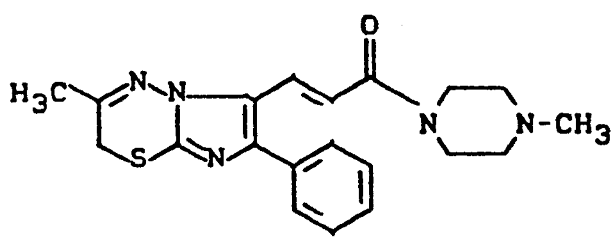

3,0 g (3-Methyl-7-phenyl-2H-imidazo[2,1-b]-1,3,4-thiadiazin-6-yl)propensäure werden in 50 ml Toluol bei 60-70° C tropfenweise mit 2,1 ml Thionylchlorid versetzt. Dann erhitzt man die Reaktionsmischung 2 h im Rückfluß und kühlt auf Raumtemperatur ab. Nun werden 6.0 g N-Methylpiperazin zugetropft und 1 h im Rückfluß erhitzt. Nach Abkühlen wird

abgesaugt und der Niederschlag mit $H_2O$ angerieben. Der wäßrige Auszug wird mit verdünnter NaOH alkalisch gestellt, abgesaugt, und der Rückstand in $CHCl_3$ aufgenommen. Die organische Phase wird mit Wasser gewaschen, getrocknet und die Lösung im Vakuum eingeengt. Der Rückstand kristallisiert mit Essigester.

Ausbeute: 26 % der Theorie

Schmelzpunkt: 195° C.

Analog Beispiel 3 und 4 wurden folgende Verbindungen hergestellt:

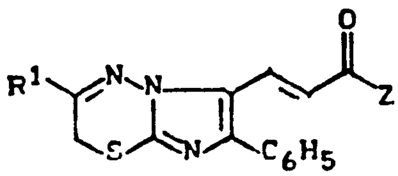

| Bsp.-Nr. | $R^1$ | Z | Schmelzpunkt [°C] |
|---|---|---|---|
| 5 | $CH_3$ | 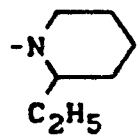 | 162 |
| 6 | $CH_3$ | 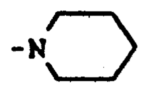 | 194 |
| 7 | $CH_3$ | $-N(C_2H_5)_2$ | 168 |
| 8 | $CH_3$ | $-NH-C_4H_9-n$ | 142 |
| 9 | $CH_3$ | $-NHCH_2CH_2OH$ | 244 |
| 10 | $CH_3$ | 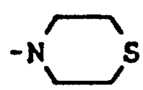 | 206 |
| 11 | $CH_3$ | 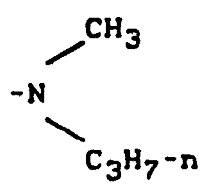 | |
| 12 | $CH_3$ | $-N(CH_2CH_2OH)_2$ | |

Patentansprüche

1. Imidazothiadiazinalkensäureamide der allgemeinen Formel (I)

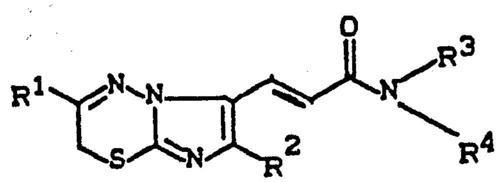

(I)

in welcher

$R^1$
- für Wasserstoff oder
- für Alkoxy oder
- für gegebenenfalls durch Phenyl, Cyano, Hydroxy oder Halogen substituiertes Alkyl oder Alkenyl steht, wobei die Kohlenstoffkette des Alkylrestes gegebenenfalls durch O, S, NH, N-Alkyl, N-Aryl oder N-Aralkyl unterbrochen ist, oder
- für einen Aryl- oder Heterocyclylrest steht, der gegebenenfalls ein, zwei oder drei gleiche oder verschiedene Substituenten aus der Gruppe Alkyl, Alkoxy, Alkylthio, Trifluormethyl, Trifluormethoxy, Halogen, Nitro, Cyano oder Alkylsulfonyl trägt, oder
- für ein Gruppe $SO_n$-Alkyl steht, worin n 0, 1 oder 2 bedeutet, oder
- für eine Gruppe der Formel

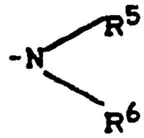 oder 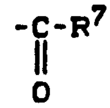 steht,

wobei

$R^5$, $R^6$ - gleich oder verschieden sind und

- für Wasserstoff, oder
- für gegebenenfalls durch O, S, NH oder N-Alkyl in der Kette unterbrochenes Alkyl stehen, oder
- für Aryl oder Aralkyl stehen,

oder

$R^5$ und $R^6$ gemeinsam mit dem Stickstoffatom einen 5 bis 7-gliedrigen Ring bilden, der gegebenenfalls ein oder zwei Sauerstoff-, Schwefel- und/oder Stickstoffatome als weitere Heteroatome enthalten kann, wobei diese Stickstoffatome gegebenenfalls durch Alkyl, Aryl oder Aralkyl substituiert sind,

und wobei

$R^7$ - für Wasserstoff,

- für Hydroxy,
- für Alkyl oder Alkenyl,
- für Alkoxy oder Alkenyloxy,
- für Aryloxy oder Aralkoxy steht, oder
- für eine Gruppe der Formel

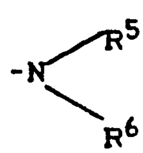 steht,

wobei $R^5$ und $R^6$ die bereits angegebene Bedeutung haben,

$R^2$ - für Aryl steht, das gegebenenfalls bis zu dreifach gleich oder verschieden substituiert ist durch Halogen, Alkyl, Alkoxy, Alkylthio, Nitro, Cyano oder Trifluormethyl, oder
- für gegebenenfalls durch Halogen, Alkyl, Alkoxy oder Trifluormethyl substituiertes Pyridyl, Thienyl, Furyl, Pyrimidyl, Pyridazinyl, Chinolyl oder Isochinolyl steht,

und

$R^3$, $R^4$ - gleich oder verschieden sind und
- für Wasserstoff,
- für Aryl, oder
- für einen geradkettigen, verzweigten oder cyclischen gesättigten oder ungesättigten Kohlenstoffrest mit bis zu 10 C-Atomen stehen der gegebenenfalls durch O, S, NH, N-Alkyl, N-Aryl oder N-Aralkyl unterbrochen ist, und der gegebenenfalls substituiert ist durch Hydroxy, Alkoxy, Trifluormethyl, Halogen, Phenyl, Alkoxycarbonyl oder Dialkylamino, wobei die beiden Alkylreste gegebenenfalls gemeinsam mit dem Stickstoffatom einen 5-7-gliedrigen Ring bilden, der gegebenenfalls durch O, S, NH oder N-Alkyl unterbrochen ist und wobei die vorgenannten Alkyl- sowie Phenylreste ihrerseits gegebenenfalls durch Halogen, Trifluormethyl, Alkyl,

Aryl, Aralkyl, Alkoxy, Alkylthio, Alkylsulfonyl oder Cyano substituiert sind,

oder

$R^3$ und $R^4$ gemeinsam mit dem Stickstoffatom einen 3- bis 8-gliedrigen, gesättigten oder ungesättigten Ring bilden, der gegebenenfalls ein oder zwei O, S, N, NH, N-Alkyl, N-Aryl oder N-Aralkyl enthält, der gegebenenfalls bis zu drei Substituenten aus der Reihe Alkyl, Halogen, Aryl, Aralkyl, Alkoxycarbonyl, Hydroxyalkyl, Alkoxyalkyl oder Trifluormethyl tragen kann, oder der mit einem gegebenenfalls substituierten aromatischen Ring kondensiert sein kann,

sowie ihre physiologisch unbedenklichen Salze, sowie ihre stereoisomeren Formen in Form von Enantiomeren, Diastereomeren und E/Z-Isomeren.

2. Imidazothiadiazinalkensäureamide der allgemeinen Formel (I) in Anspruch 1,

in welcher

$R^1$ - für Wasserstoff oder
- für $C_1$-$C_6$ Alkoxy steht, oder
- für gegebenenfalls durch Phenyl, Cyano, Fluor, Chlor oder Brom, Hydroxy substituiertes $C_1$-$C_6$-Alkyl oder $C_2$-$C_6$-Alkenyl, Cyclopentyl, Cyclohexyl steht, wobei die Kohlenstoffkette des

Alkylrestes gegebenenfalls durch O, S, NH, N-$C_1$-$C_6$-Alkyl, N-Phenyl oder N-Benzyl unterbrochen ist, oder

- für einen Phenyl-, Naphthyl-, Furyl-, Thienyl-, Pyrimidyl-, Pyrazinyl-, Pyridyl-, Chinolyl- oder Isochinolylrest steht, der gegebenenfalls durch ein oder zwei gleiche oder verschiedene Substituenten der Reihe, $C_1$-$C_6$-Alkyl, $C_1$-$C_6$-Alkoxy, Fluor, Chlor, Brom, Nitro, Cyano, Trifluormethyl, Trifluormethoxy oder $C_1$-$C_6$-Alkylsulfonyl substituiert ist, oder
- für eine Gruppe $SO_n$-Alkyl steht,

wobei

n für 0 oder 2 steht und Alkyl bis zu 6 C-Atome hat,

oder

- für eine Gruppe der Formel

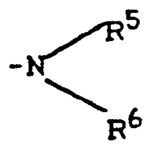 oder 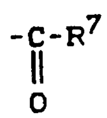 steht,

wobei

$R^5$, $R^6$ - gleich oder verschieden sind und
- für Wasserstoff stehen, oder
- für $C_1$-$C_6$-Alkyl stehen, das gegebenenfalls durch O, S, NH, oder N-$C_1$-$C_6$-Alkyl unterbrochen ist, oder
- für Phenyl, Naphthyl oder Benzyl stehen,

oder

$R^5$ und $R^6$ gemeinsam mit dem Stickstoffatom einen 5 bis 7-gliedrigen Ring bilden, der gegebenenfalls ein oder zwei Sauerstoff-, Schwefel- und/oder Stickstoffatome als weitere Heteroatome enthalten kann, und wobei diese Stickstoffatome gegebenenfalls durch $C_1$-$C_6$-Alkyl, Phenyl oder Benzyl substituiert sind,

und wobei

$R^7$ - für Wasserstoff,
- für Hydroxy,
- für $C_1$-$C_6$-Alkyl, $C_2$-$C_6$-Alkenyl,
- für $C_1$-$C_6$-Alkoxy, $C_2$-$C_6$-Alkenyloxy,
- für Phenoxy oder Benzyloxy steht, oder
- für eine Gruppe der Formel

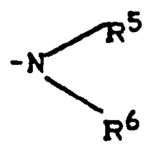

steht,

wobei

$R^5$ und $R^6$ die bereits angegebene Bedeutng haben,

$R^2$ - für gegebenenfalls durch Fluor, Chlor, Brom, $C_1$-$C_6$-Alkyl, $C_1$-$C_6$-Alkoxy, Nitro, Cyano oder Trifluormethyl substituiertes Phenyl steht, oder
- für gegebenenfalls durch Fluor, Chlor, Brom oder $C_1$-$C_4$-Alkyl substituiertes Pyridyl, Thienyl, Furyl, oder Pyrimidyl steht,

und

$R^3$, $R^4$ - gleich oder verschieden sind und
- für Wasserstoff,
- für Phenyl oder

- für einen geradkettigen, verzweigten oder cyclischen Kohlenwasserstoffrest mit bis zu 8 C-Atomen stehen, der gegebenenfalls durch O, S, NH, N-$C_1$-$C_6$-Alkyl, N-Phenyl oder N-Benzyl unterbrochen ist, und der gegebenenfalls substituiert ist durch Hydroxy, $C_1$-$C_6$-Alkoxy, Trifluormethyl, Fluor, Chlor, Brom, Phenyl, $C_1$-$C_6$-Alkoxycarbonyl, Di-$C_1$-$C_6$-Alkylamino, Piperidin, Piperazin, Morpholin, Thiomorpholin, N-Methylpiperazin oder Pyrrolidin, wobei die vorgenannten Alkyl-sowie Phenylreste ihrerseits gegebenenfalls durch Fluor, Chlor, Brom, Trifluormethyl, $C_1$-$C_6$-Alkyl, Phenyl, Benzyl, $C_1$-$C_6$-Alkoxy, $C_1$-$C_6$-Alkylsulfonyl oder Cyano substituiert sind,

oder

$R^3$ und $R^4$ gemeinsam mit dem Stickstoffatom einen 5- bis 7-gliedrigen Ring bilden, der gegebenenfalls 1 oder 2 weitere Heteroatome aus der Gruppe Sauerstoff, Schwefel oder Stickstoff enthält, wobei der Stickstoff gegebenenfalls durch Wasserstoff, $C_1$-$C_6$-Alkyl, Phenyl oder Benzyl substituiert ist und wobei der Ring durch bis zu drei gleiche oder verschiedene Substituenten aus der Reihe $C_1$-$C_6$-Alkyl, Fluor, Chlor, Brom, Phenyl, Benzyl, $C_1$-$C_6$-Alkoxycarbonyl, Hydroxy-$C_1$-$C_6$-alkyl, $C_1$-$C_4$-Alkoxy-$C_1$-$C_6$-alkyl oder Trifluormethyl substituiert sein kann, oder wobei der Ring mit einem gegebenenfalls

durch Fluor, Chlor, Brom oder Nitro substituierten aromatischen Ring kondensiert sein kann,

sowie ihre physiologisch unbedenklichen Salze, sowie ihre stereoisomeren Formen in Form von Enantiomeren, Diastereomeren und E/Z-Isomeren.

3. Imidazothiadiazinalkensäureamide der allgemeinen Formel (I) in Anspruch 1,

in welcher

$R^1$ - für Wasserstoff,
- für geradkettiges oder verzweigtes $C_1$-$C_4$-Alkoxy oder
- für gegebenenfalls durch Fluor, Chlor oder Hydroxy substituiertes geradkettiges oder verzweigtes $C_1$-$C_6$-Alkyl steht, wobei die Alkylkette gegebenenfalls durch O, S, NH, N-$C_1$-$C_4$-Alkyl, N-Phenyl oder N-Benzyl unterbrochen ist,

oder

- für einen Phenyl-, Naphthyl-, Furyl-, Thienyl-, Pyrimidyl- oder Pyridylrest steht, oder
- für eine Gruppe der Formel

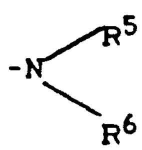 oder 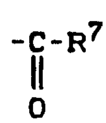 steht

wobei

$R^5$, $R^6$ - gleich oder verschieden sind und

- für Wasserstoff oder
- für geradkettiges oder verzweigtes $C_1$-$C_6$-Alkyl stehen, das gegebenenfalls durch O, S, NH oder N-$C_1$-$C_3$-Alkyl unterbrochen ist, oder
- für Phenyl oder Benzyl stehen

oder

$R^5$ und $R^6$ gemeinsam mit dem Stickstoffatom einen 5-bis 6-gliedrigen Ring bilden, der ein Sauerstoff-, Schwefel- und/oder Stickstoffatom als weiteres Heteroatom enthalten kann, und wobei dieses Stickstoffatom gegebenenfalls durch $C_1$-$C_3$-Alkyl, Phenyl oder Benzyl substituiert ist,

und wobei

$R^7$ - für Wasserstoff,

- für Hydroxy,
- für geradkettiges oder verzweigtes $C_1$-$C_4$-Alkyl,
- für geradkettiges oder verzweigtes $C_1$-$C_4$-Alkoxy oder
- für Phenoxy oder Benzyloxy steht,

$R^2$ - für gegebenenfalls durch Chlor, Brom oder $C_1$-$C_4$-Alkyl substituiertes Phenyl, oder

- für Thienyl steht,

und

$R^3$, $R^4$ - gleich oder verschieden sind und
- für Wasserstoff,
- für Phenyl oder
- für einen geradkettigen, verzweigten oder cyclischen Kohlenwasserstoffrest mit bis zu 6 C-Atomen stehen, der gegebenenfalls durch O, S, NH, N-$C_1$-$C_4$-Alkyl, N-Phenyl oder N-Benzyl unterbrochen ist, und der gegebenenfalls substituiert ist durch Hydroxy, $C_1$-$C_4$-Alkoxy, Trifluormethyl, Fluor, Chlor, Phenyl, Di-$C_1$-$C_4$-alkylamino, Piperidin, Piperazin, N-Methylpiperazin, Morpholin oder Pyrrolidin, wobei die vorgenannten Alkyl- oder Phenylreste ihrerseits gegebenenfalls durch Fluor, Chlor, Trifluormethyl, $C_1$-$C_4$-Alkyl oder $C_1$-$C_4$-Alkoxy substituiert sind,

oder

$R^3$ und $R^4$ gemeinsam mit dem Stickstoffatom einen 5- bis7-gliedrigen Ring bilden, der gegebenenfalls 1 oder zwei weitere Heteroatome aus der Gruppe Sauerstoff, Schwefel oder Stickstoff enthält, wobei der Stickstoff gegebenenfalls durch Wasserstoff, $C_1$-$C_4$-Alkyl, Phenyl oder Benzyl substituiert ist und wobei der Ring durch bis zu zwei gleiche oder verschiedene Substituenten aus der Reihe $C_1$-$C_4$-Alkyl, Fluor, Chlor, $C_1$-$C_4$-Hydroxyalkyl, $C_1$-$C_4$-Alkoxy-$C_1$-$C_4$-alkyl oder

Trifluormethyl substituiert sein kann oder wobei der Ring mit einem aromatischen Ring kondensiert sein kann,

sowie ihre physiologisch unbedenklichen Salze, sowie ihre stereoisomeren Formen in Form von Enantiomeren, Diastereomeren und E/Z-Isomeren.

4. Imidazothiadiazinalkensäureamide der allgemeinen Formel (I)

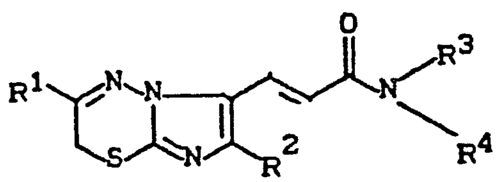

(I)

in welcher

$R^1$ - für Wasserstoff oder

- für Alkoxy oder
- für gegebenenfalls durch Phenyl, Cyano, Hydroxy oder Halogen substituiertes Alkyl oder weitere Heteroatome enthalten kann, wobei der Stickstoff gegebenenfalls durch Alkyl, Aryl oder Aralkyl substituiert ist, oder
- für einen Aryl- oder Heterocyclylrest steht, der gegebenenfalls ein, zwei oder drei gleiche oder verschiedene Substituenten aus der Gruppe Alkyl, Alkoxy, Alkylthio, Trifluormethyl, Trifluormethoxy, Halogen, Nitro, Cyano oder Alkylsulfonyl trägt, oder
- für ein Gruppe $SO_n$-Alkyl steht, worin n 0, 1 oder 2 bedeutet, oder
- für eine Gruppe der Formel

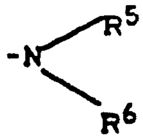 oder 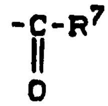 steht,

wobei

$R^5$, $R^6$ - gleich oder verschieden sind und
- für Wasserstoff, oder
- für gegebenenfalls durch O, S, NH oder N-Alkyl in der Kette unterbrochenes Alkyl stehen, oder
- für Aryl oder Aralkyl stehen,

oder

$R^5$ und $R^6$ gemeinsam mit dem Stickstoffatom einen 5 bis 7-gliedrigen Ring bilden, der gegebenenfalls ein oder zwei Sauerstoff-, Schwefel- und/oder Stickstoffatome als weitere Heteroatome enthalten kann und wobei diese stickstoffatome gegebenenfalls durch Alkyl, Aryl oder Aralkyl substituiert sind,

und wobei

$R^7$ - für Wasserstoff,
- für Hydroxy,
- für Alkyl oder Alkenyl,
- für Alkoxy oder Alkenyloxy,
- für Aryloxy oder Aralkoxy steht, oder
- für eine Gruppe der Formel

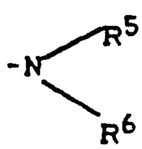 steht,

wobei

$R^5$ und $R^6$ die bereits angegebene Bedeutung haben,

$R^2$ - für Aryl steht, das gegebenenfalls bis zu dreifach gleich oder verschieden substituiert ist durch Halogen, Alkyl, Alkoxy, Alkylthio, Nitro, Cyano oder Trifluormethyl, oder

- für gegebenenfalls durch Halogen, Alkyl, Alkoxy oder Trifluormethyl substituiertes Pyridyl, Thienyl, Furyl, Pyrimidyl, Pyridazinyl, Chinolyl oder Isochinolyl steht,

und

$R^3$, $R^4$ - gleich oder verschieden sind und

- für Wasserstoff,
- für Aryl, oder
- für einen geradkettigen, verzweigten oder cyclischen gesättigten oder ungesättigten Kohlenwasserstoffrest mit bis zu 10 C-Atomen steht, der gegebenenfalls substituiert ist durch Hydroxy, Alkoxy, Trifluormethyl, Halogen, Phenyl, Alkoxycarbonyl oder Dialkylamino, wobei die beiden Alkylreste gegebenenfalls gemeinsam mit dem Stickstoffatom einen 5-7-gliedrigen Ring bilden, der gegebenenfalls durch O, S, NH oder N-Alkyl unterbrochen ist und wobei die vorgenannten Alkyl- sowie Phenylreste ihrerseits gegebenenfalls durch Halogen, Trifluormethyl, Alkyl, Aryl, Aralkyl, Alkoxy, Alkylthio, Alkylsulfonyl oder Cyano substituiert sind,

oder

$R^3$ und $R^4$ gemeinsam mit dem Stickstoffatom einen 3- bis 8-gliedrigen, gesättigten oder ungesättigten Ring bilden, der gegebenenfalls ein oder zwei O, S, N, NH, N-Alkyl, N-Aryl oder N-Aralkyl enthält, der gegebenenfalls bis zu drei Substituenten aus der Reihe Alkyl, Halogen, Aryl, Aralkyl, Alkoxycarbonyl, Hydroxyalkyl, Alkoxyalkyl oder Trifluormethyl tragen kann, oder der mit einem gegebenenfalls substituierten aromatischen Ring kondensiert sein kann,

sowie ihre physiologisch unbedenklichen Salze, sowie ihre stereoisomeren Formen in Form von Enantiomeren, Diastereomeren und E/Z-Isomeren bei der Verwendung zur Behandlung von Krankheiten.

5. Verfahren zur Herstellung von Imidazothiadiazinalkensäureamiden der allgemeinen Formel (I)

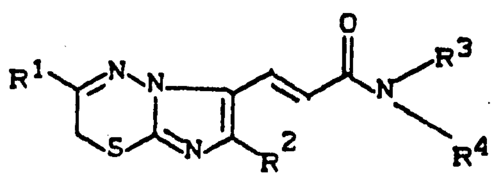

(I)

in welcher

$R^1$
- für Wasserstoff oder
- für Alkoxy oder
- für gegebenenfalls durch Phenyl, Cyano, Hydroxy oder Halogen substituiertes Alkyl oder Alkenyl steht, wobei die Kohlenstoffkette des

Alkylrestes gegebenenfalls durch O, S, NH, N-Alkyl, N-Aryl oder N-Aralkyl unterbrochen ist, oder

- für einen Aryl- oder Heterocyclylrest steht, der gegebenenfalls ein, zwei oder drei gleiche oder verschiedene Substituenten aus der Gruppe Alkyl, Alkoxy, Alkylthio, Trifluormethyl, Trifluormethoxy, Halogen, Nitro, Cyano oder Alkylsulfonyl trägt, oder
- für ein Gruppe $SO_n$-Alkyl steht, worin

n 0, 1 oder 2 bedeutet, oder

- für eine Gruppe der Formel

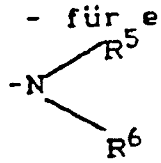 oder 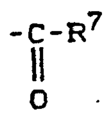 steht,

wobei

$R^5$, $R^6$ - gleich oder verschieden sind und
- für Wasserstoff, oder
- für gegebenenfalls durch O, S, NH oder N-Alkyl in der Kette unterbrochenes Alkyl stehen, oder
- für Aryl oder Aralkyl stehen,

oder

$R^5$ und $R^6$ gemeinsam mit dem Stickstoffatom einen 5 bis 7-gliedrigen Ring bilden, der gegebenenfalls ein oder zwei Sauerstoff-, Schwefel- und/oder Stickstoffatome als weitere Heteroatome enthalten kann und wobei diese Stickstoffatome gegebenenfalls durch Alkyl, Aryl oder Aralkyl substituiert sind,

und wobei

$R^7$
- für Wasserstoff,
- für Hydroxy,
- für Alkyl oder Alkenyl,
- für Alkoxy oder Alkenyloxy,
- für Aryloxy oder Aralkoxy steht, oder
- für eine Gruppe der Formel

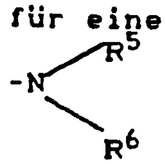 steht,

wobei $R^5$ und $R^6$ die bereits angegebene Bedeutung haben,

$R^2$
- für Aryl steht, das gegebenenfalls bis zu dreifach gleich oder verschieden substituiert ist durch Halogen, Alkyl, Alkoxy, Alkylthio, Nitro, Cyano oder Trifluormethyl, oder
- für gegebenenfalls durch Halogen, Alkyl, Alkoxy oder Trifluormethyl substituiertes Pyridyl, Thienyl, Furyl, Pyrimidyl, Pyridazinyl, Chinolyl oder Isochinolyl steht,

und

$R^3$, $R^4$ - gleich oder verschieden sind und
- für Wasserstoff,
- für Aryl, oder
- für einen geradkettigen, verzweigten oder cyclischen gesättigten oder ungesättigten Kohlenwasserstoffrest mit bis zu 10 C-Atomen stehen der gegebenenfalls durch O, S, NH, N-Alkyl, N-Aryl oder N-Aralkyl unterbrochen ist, und der gegebenenfalls substituiert ist durch Hydroxy, Alkoxy, Trifluormethyl, Halogen, Phenyl, Alkoxycarbonyl oder Dialkylamino, wobei die beiden Alkylreste gegebenenfalls gemeinsam mit dem Stickstoffatom einen 5-7-gliedrigen Ring bilden, der gegebenenfalls durch O, S, NH oder N-Alkyl

unterbrochen ist und wobei die vorgenannten Alkyl- sowie Phenylreste ihrerseits gegebenenfalls durch Halogen, Trifluormethyl, Alkyl, Aryl, Aralkyl, Alkoxy, Alkylthio, Alkylsulfonyl oder Cyano substituiert sind,

oder

$R^3$ und $R^4$ gemeinsam mit dem Stickstoffatom einen 3- bis 8-gliedrigen, gesättigten oder ungesättigten Ring bilden, der gegebenenfalls ein oder zwei O, S, N, NH, N-Alkyl, N-Aryl oder N-Aralkyl enthält, der gegebenenfalls bis zu drei Substituenten aus der Reihe Alkyl, Halogen, Aryl, Aralkyl, Alkoxycarbonyl, Hydroxyalkyl, Alkoxyalkyl oder Trifluormethyl tragen kann, oder der mit einem gegebenenfalls substituierten aromatischen Ring kondensiert sein kann,

sowie ihren physiologisch unbedenklichen Salzen, sowie ihre stereoisomeren Formen in Form von Enantiomeren, Diastereomeren und E/Z-Isomeren, dadurch gekennzeichnet, daß man

[A] Aldehyde der allgemeinen Formel (II)

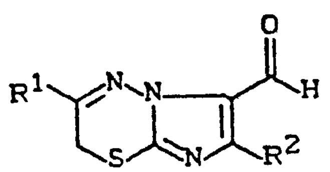

(II),

in welcher

$R^1$, $R^2$ die bereits angegebene Bedeutung haben, mit Phosphonaten der allgemeinen Formel (III)

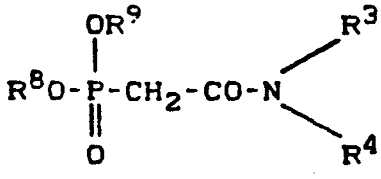

(III),

in welcher

$R^3$, $R^4$ die bereits angegebene Bedeutung haben,
$R^8$, $R^9$ für gegebenenfalls substituiertes Alkyl oder Aralkyl stehen,

in Gegenwart von starken Basen in inerten organischen Lösungsmitteln bei Temperaturen von -20° C bis +150° C, bevorzugt von -20° C bis +110° C umsetzt,

oder daß man

[B] Aldehyde der allgemeinen Formel (II) mit Acetamiden der allgemeinen Formel (IV)

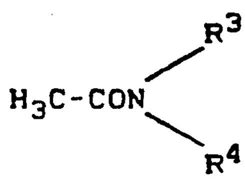

(IV),

in welcher

$R^3$, $R^4$ die bereits angegebene Bedeutung haben, in Gegenwart saurer oder basicher Katalysatoren, gegebenenfalls in Anwesenheit inerter organischer Lösungsmittel bei Temperaturen zwischen 0° C und +200° C, bevorzugt zwischen +20° C und +150° C umsetzt,

oder daß man

[C] Alkencarbonsäuren der allgemeinen Formel (V)

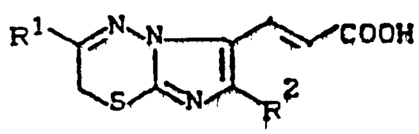

(V)

in welcher

$R^1$, $R^2$ die bereits angegebene Bedeutung haben, mit Aminen der allgemeinen Formel (VI)

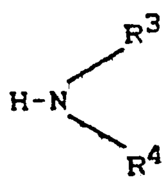

(VI),

in welcher

$R^3$, $R^4$ die bereits angegebene Bedeutung haben, gegebenenfalls nach Aktivierung der Carboxylgruppe über das entsprechende Säurechlorid in inerten organischen Lösungsmitteln bei Temperaturen zwischen 0°C und 150°C, bevorzugt zwischen 20°C und 100°C umsetzt.

6. Aldehyde der allgemeinen Formel (II)

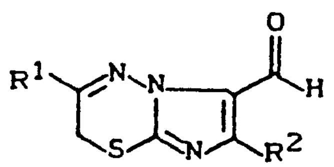

(II),

in welcher

$R^1$, $R^2$ die bereits in Anspruch 5 angegebene Bedeutung haben.

7. Verfahren zur Herstellung von Aldehyden der allgemeinen Formel (II)

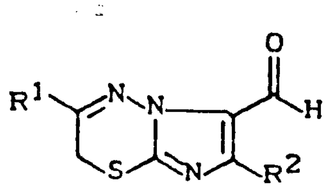

(II),

in welcher

$R^1$ - für Wasserstoff oder
- für Alkoxy oder

- für gegebenenfalls durch Phenyl, Cyano, Hydroxy oder Halogen substituiertes Alkyl oder Alkenyl steht, wobei die Kohlenstoffkette des Alkylrestes gegebenenfalls durch O, S, NH, N-Alkyl, N-Aryl oder N-Aralkyl unterbrochen ist, oder
- für einen Aryl- oder Heterocyclylrest steht, der gegebenenfalls ein, zwei oder drei gleiche oder verschiedene Substituenten aus der Gruppe Alkyl, Alkoxy, Alkylthio, Trifluormethyl, Trifluormethoxy, Halogen, Nitro, Cyano oder Alkylsulfonyl trägt, oder
- für ein Gruppe $SO_n$-Alkyl steht, worin n 0, 1 oder 2 bedeutet, oder
- für eine Gruppe der Formel

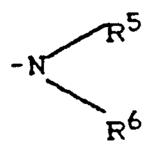 oder 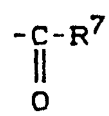 steht,

wobei

$R^5$, $R^6$ - gleich oder verschieden sind und
- für Wasserstoff, oder
- für gegebenenfalls durch O, S, NH oder N-Alkyl in der Kette unterbrochenes Alkyl stehen, oder
- für Aryl oder Aralkyl stehen,

oder

$R^5$ und $R^6$ gemeinsam mit dem Stickstoffatom einen 5 bis 7-gliedrigen Ring bilden, der gegebenenfalls ein oder zwei

Sauerstoff-, Schwefel- und/oder Stickstoffatome als weitere Heteroatome enthalten kann und wobei diese Stickstoffatome gegebenenfalls durch Alkyl, Aryl oder Aralkyl substituiert sind,

und wobei

$R^7$ - für Wasserstoff,
- für Hydroxy,
- für Alkyl oder Alkenyl,
- für Alkoxy oder Alkenyloxy,
- für Aryloxy oder Aralkoxy steht, oder
- für eine Gruppe der Formel

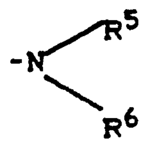

steht,

wobei

$R^5$ und $R^6$ die bereits angegebene Bedeutung haben,

$R^2$ - für Aryl steht, das gegebenenfalls bis zu dreifach gleich oder verschieden substituiert ist durch Halogen, Alkyl, Alkoxy, Alkylthio, Nitro, Cyano oder Trifluormethyl, oder
- für gegebenenfalls durch Halogen, Alkyl, Alkoxy oder Trifluormethyl substituiertes Pyridyl, Thienyl, Furyl, Pyrimidyl, Pyridazinyl, Chinolyl oder Isochinolyl steht,

dadurch gekennzeichnet, daß man Imidazothiadiazine der allgemeinen Formel (VII)

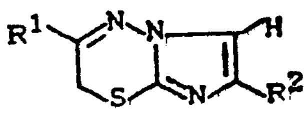

(VII),

in welcher

$R^1$ und $R^2$ die angegebene Bedeutung haben, mit Phosphoroxychlorid und Dimethylformamid bei Temperaturen von 0°C bis + 150 °C umsetzt.

8. Alkencarbonsäuren der allgemeinen Formel (V)

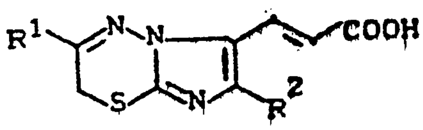

(V)

in welcher

$R^1$ - für Wasserstoff oder
- für Alkoxy oder
- für gegebenenfalls durch Phenyl, Cyano, Hydroxy oder Halogen substituiertes Alkyl oder Alkenyl steht, wobei die Kohlenstoffkette des Alkylrestes gegebenenfalls durch O, S, NH, N-Alkyl, N-Aryl oder N-Aralkyl unterbrochen ist, oder
- für einen Aryl- oder Heterocyclylrest steht, der gegebenenfalls ein, zwei oder drei gleiche oder verschiedene Substituenten aus der Gruppe

Alkyl, Alkoxy, Alkylthio, Trifluormethyl, Trifluormethoxy, Halogen, Nitro, Cyano oder Alkylsulfonyl trägt, oder

- für ein Gruppe $SO_n$-Alkyl steht,

  worin

  n 0, 1 oder 2 bedeutet, oder

- für eine Gruppe der Formel

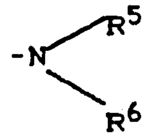 oder 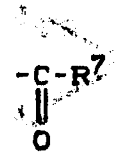 steht,

wobei

$R^5$, $R^6$ - gleich oder verschieden sind und

- für Wasserstoff, oder
- für gegebenenfalls durch O, S, NH oder N-Alkyl in der Kette unterbrochenes Alkyl stehen, oder
- für Aryl oder Aralkyl stehen,

oder

$R^5$ und $R^6$ gemeinsam mit dem Stickstoffatom einen 5 bis 7-gliedrigen Ring bilden, der gegebenenfalls ein oder zwei Sauerstoff-, Schwefel- und/oder Stickstoffatome als weitere Heteroatome enthalten kann, wobei diese Stickstoffatome gegebenenfalls durch Alkyl, Aryl oder Aralkyl substituiert sind,

und wobei

$R^7$ - für Wasserstoff,
- für Hydroxy,
- für Alkyl oder Alkenyl,
- für Alkoxy oder Alkenyloxy,
- für Aryloxy oder Aralkoxy steht, oder
- für eine Gruppe der Formel

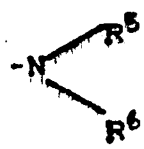

steht,

wobei

$R^5$ und $R^6$ die bereits angegebene Bedeutung haben

$R^2$ - für Aryl steht, das gegebenenfalls bis zu dreifach gleich oder verschieden substituiert ist durch Halogen, Alkyl, Alkoxy, Alkylthio, Nitro, Cyano oder Trifluormethyl, oder
- für gegebenenfalls durch Halogen, Alkyl, Alkoxy oder Trifluormethyl substituiertes Pyridyl, Thienyl, Furyl, Pyrimidyl, Pyridazinyl, Chinolyl oder Isochinolyl steht,

9. Verfahren zur Herstellung von Alkencarbonsäuren der allgemeinen Formel (V)

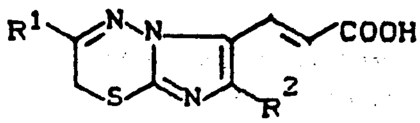

(V)

in welcher

$R^1$ - für Wasserstoff oder

- für Alkoxy oder
- für gegebenenfalls durch Phenyl, Cyano, Hydroxy oder Halogen substituiertes Alkyl oder Alkenyl steht, wobei die Kohlenstoffkette des Alkylrestes gegebenenfalls durch O, S, NH, N-Alkyl, N-Aryl oder N-Aralkyl unterbrochen ist, oder
- für einen Aryl- oder Heterocyclylrest steht, der gegebenenfalls ein, zwei oder drei gleiche oder verschiedene Substituenten aus der Gruppe Alkyl, Alkoxy, Alkylthio, Trifluormethyl, Trifluormethoxy, Halogen, Nitro, Cyano oder Alkylsulfonyl trägt, oder
- für ein Gruppe $SO_n$-Alkyl steht, worin n 0, 1 oder 2 bedeutet, oder
- für eine Gruppe der Formel

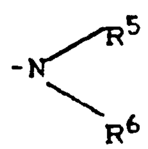

oder

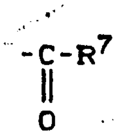

steht,

wobei

$R^5$, $R^6$ - gleich oder verschieden sind und
- für Wasserstoff, oder
- für gegebenenfalls durch O, S, NH oder N-Alkyl in der Kette unterbrochenes Alkyl stehen, oder
- für Aryl oder Aralkyl stehen,

oder

$R^5$ und $R^6$ gemeinsam mit dem Stickstoffatom einen 5 bis 7-gliedrigen Ring bilden, der gegebenenfalls ein oder zwei Sauerstoff-, Schwefel- und/oder Stickstoffatome als weitere Heteroatome enthalten kann, wobei der Stickstoff gegebenenfalls durch Alkyl, Aryl oder Aralkyl substituiert ist,

und wobei

$R^7$ - für Wasserstoff,
- für Hydroxy,
- für Alkyl oder Alkenyl,
- für Alkoxy oder Alkenyloxy,
- für Aryloxy oder Aralkoxy steht, oder
- für eine Gruppe der Formel

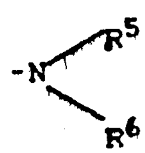

steht,

wobei
$R^5$ und $R^6$ die bereits angegebene Bedeutung haben,

$R^2$ - für Aryl steht, das gegebenenfalls bis zu dreifach gleich oder verschieden substituiert ist durch Halogen, Alkyl, Alkoxy, Alkylthio, Nitro, Cyano oder Trifluormethyl, oder

- für gegebenenfalls durch Halogen, Alkyl, Alkoxy oder Trifluormethyl substituiertes Pyridyl, Thienyl, Furyl, Pyrimidyl, Pyridazinyl, Chinolyl oder Isochinolyl steht,

dadurch gekennzeichnet, daß man

[X] Carbonylverbindungen der allgemeinen Formel (II) mit Phosphonaten der allgemeinen Formel (VIII)

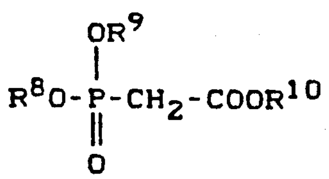

(VIII),

in welcher

$R^8$, $R^9$ - für gegebenenfalls substituiertes Alkyl oder Aralkyl stehen,

$R^{10}$ - für Wasserstoff, Trialkylsilyl, Triarylsilyl oder für Alkyl oder Aralkyl steht,

in Gegenwart starker Basen in inerten organischen Lösungsmitteln zunächst zu den entsprechenden Alkensäurederivaten umsetzt und diese dann mit Säuren oder Basen zu den freien Carbonsäuren verseift,

oder indem man

[Y] Aldehyde der allgemeinen Formel (II) mit Malonsäure ($H_2C(COOH)_2$) oder mit Meldrumsäure in Gegenwart inerter organischer Lösungsmittel, gegebenenfalls in Gegenwart von Kondensationsmitteln umsetzt.

10. Arzneimittel, gekennzeichnet, durch einen Gehalt an mindestens einem Imidazothiadiazinalkensäureamid der allgemeinen Formel (I) in Anspruch 1 oder deren physiologisch unbedenklichen Salzen.

11. Verwendung von Verbindungen gemäß Anspruch 1 bei der Bekämpfung von Krankheiten.

0224809

Europäisches Patentamt

# EUROPÄISCHER RECHERCHENBERICHT

Nummer der Anmeldung

EP 86 11 6122

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| D,A | EP-A-0 041 215 (BAYER) * Ansprüche 1,7; Seite 18, Zeilen 1-13 * & US-A-4 444 770 ----- | 1,10 | C 07 D 513/04<br>A 61 K 31/54 //<br>(C 07 D 513/04<br>C 07 D 285:00<br>C 07 D 235:00 ) |
| | | | **RECHERCHIERTE SACHGEBIETE (Int. Cl 4)** |
| | | | C 07 D 513/00<br>A 61 K 31/00 |

Der vorliegende Recherchenbericht wurde für alle Patentansprüche erstellt.

| Recherchenort | Abschlußdatum der Recherche | Prüfer |
|---|---|---|
| DEN HAAG | 10-03-1987 | ALFARO I. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument